# EUROPEAN PATENT SPECIFICATION

(11) **EP 0 580 385 B1**
(45) Date of publication and mention of the grant of the patent: **08.05.1996**
(21) Application number: 93305649.1
(22) Date of filing: 19.07.1993
(51) Int. Cl.: A61M 16/00, A61B 5/00

(54) **Laryngeal mask incorporating a reflectance oximeter**
Maske für den Kehlkopf mit einem integrierten Reflexionsoximeter
Masque pour le larynx avec un oximètre à réflexion incorporé

(30) Priority: 21.07.1992 GB 9215455; 23.11.1992 US 980581
(43) Date of publication of application: 26.01.1994
(73) Proprietor: Brain, Archibald Ian Jeremy, Dr., Bourne End Buckinghamshire SL8 5DL (GB)
(72) Inventor: Brain, Archibald Ian Jeremy, Dr., Bourne End Buckinghamshire SL8 5DL (GB)
(74) Representative: West, Alan Harry

(56) References cited:
- EP-A- 0 389 272
- EP-A- 0 504 725
- FR-A- 2 517 974
- US-A- 4 765 340
- IEE TRANSACTIONS ON BIOMEDICAL ENGENEERING vol. 37, no. 6, June 1990, pages 632 - 639 WEIJIA CUI ET AL. "In Vivo Reflectance of Blood and Tissue as a Function of Light Wavelength"

## Description

This invention relates to laryngeal mask airway devices for facilitating lung ventilation in an unconscious patient and to the coordinated use therewith of a reflectance oximeter for monitoring oxygenation in the blood as an indicator of current effectiveness of the lung ventilation.

Laryngeal-mask devices have been described in my U.S. Patents No. 4,509,514 (EP-A-0 389 272) and No. 4,995,388 (FR-A-2 517 974), as well as in my U.S. patent No 5 241 956 filed September 28, 1992. EP-A-0 389 272 forms the preamble of claim 1. Such masks are artificial airway devices designed to facilitate lung ventilation in an unconscious patient by forming a low-pressure seal around the laryngeal inlet. A seal surrounds an appropriately shaped mask which fits into the lower pharynx and is attached to an airway tube which emerges from the mouth, as for connection to medical gas-supply tubing. More specifically, the mask may be a plate having peripherally sealed engagement to the laryngeal inlet; and an elongate airway tube has a distal end that is sealed to the plate, for completing an externally accessible airway passage through the plate, for exclusive airway communication with the laryngeal inlet.

Pulse oximetry is a technique for non-invasively measuring the oxygen level in blood and has proved to be a very useful clinical tool as a means of continuously assessing the adequacy of ventilation, particularly in anaesthetized patients, for whom adequate oxygenation is a key concern. Most commercial pulse oximeters measure the signal when light of appropriate wavelength is shone through a tissue such as a finger, and there are now more than 30 manufacturers of such devices. However, there are disadvantages to this technique, the most important being that the finger probe may easily become detached during use, with loss of signal, perhaps at a critical moment; also, since the device is peripheral in position, there is sometimes poor correlation between blood oxygenation in the finger, and oxygenation where it matters most -- for example, in the brain. Attempts to rectify this have resulted in development of probes for use on the ear, eyelid, and nose, with varying degrees of success.

Another approach has been to develop a related technique, reflectance oximetry, which has been described theoretically by Cui, Ostrander and Lee ("In Vivo Reflectance of Blood and Tissue as a Function of Light Wavelength", IEEE Translations on Biomedical Engineering, 1990, Vol. 37, No. 6: 632 to 639) and in certain practical applications. See, for example, in this connection: Cheng, Hopwood and Kay, "Forehead Pulse Oximetry Compared with Finger Pulse Oximetry and Arterial Blood Gas Measurement", Journal of Clinical Monitoring, 1988, Vol. 4, No. 3: 223 to 226; Mendelson and McGinn, "Skin Reflectance Pulse Oximetry: In Vivo Measurements from the Forearm and Calf", Journal of Clinical Monitoring, 1991, Vol. 7, No. 1: 7 to 12; Mendelson and Ochs, "Noninvasive Pulse Oximetry Utilising Skin Reflectance Photoplethysmography" IEEE Transactions on Biomedical Engineering, 1988, Vol. 35, No. 10: 798 to 805; and Johnson, Johnson, Fisher, Jobbings, Bannister, and Lilford, "Fetal Monitoring with Pulse Oximetry" British Journal of Obstetrics and Gynaecology, 1991, Vol. 98: 36 to 41. The aim of such procedures is to measure a signal reflected back from a tissue surface instead of having to rely on light transmission. This avoids the necessity of using peripheral tissues such as the finger, since tissue thickness is no longer a problem. The disadvantages of the reflectance oximeter are that accuracy is reduced, in that there may be problems with incident light, and it may be necessary to heat the skin in order to maximise blood flow in the skin, noting that skin is always at a lower temperature than blood.

The present invention seeks to incorporate a reflectance oximeter into a laryngeal-mask airway as to avoid the above disadvantages of conventional pulse oximetry and reflectance oximetry.

The invention achieves the above objective by so mounting a reflectance oximeter to the upstream or proximal side of a laryngeal mask as to face the posterior wall of the pharynx when the laryngeal mask has been positioned to perform its function of sealed, exclusive airway communication with the laryngeal inlet. Thus, positioned, radiation from the oximeter can utilise local back-bone features as reflector, for two-way passage of the radiation through tissue which characterises the posterior wall of the pharynx. Moreover, the oximeter-observation region is within the body so that ambient light has no degrading effect, and changes in oxygen saturation will be detected earlier than by use of any peripherally placed oximeter probe.

In accordance with the invention, there is now provided a laryngeal mask for facilitating lung ventilation, the mask having anterior and posterior sides and means comprising for establishing sealed peripheral engagement of the mask around the laryngeal inlet of a patient, with the anterior side facing into the laryngeal inlet and the posterior side facing the back wall of the pharynx, an elongate airway tube having a distal end which establishes a sealed passage through the mask between the posterior and anterior sides of the mask, and a reflectance-oximeter probe carried at the posterior side of the mask and adapted and oriented to radiate into and to sense radiation reflected from within a localised portion of the back wall of the pharynx.

In its more general aspect, the apparatus of the invention can be considered to be an artificial airway device to facilitate a patent's lung ventilation, comprising an airway tube, an evacuation tube, and a laryngeal mask at one of the of the tubes, the mask including a first inflatable-cuff formation of flexible material in a generally elliptical configuration extending from a proximal end to a distal end and in generally a single plane which is inclined to the axis of the airway tube at the distal end of the airway tube, a second inflatable-cuff formation carried by the mask on the posterior side of the plane, the mask being configured upon inflation of the cuff formations (1) to form a seal of the airway tube solely around the circumference of the laryngeal inlet and (2) to establish a cushioning action via the second cuff formation between the posterior side of the mask and the posterior wall of the pharynx, the distal end of the first cuff formation being configured for entry into and insertional location of the device by engagement with the oesophagus at the upper sphinctral region of the oesophagus when the mask is positioned for sealing the airway tube to the laryngeal inlet, the evacuation tube having an open distal end centrally within and axially short of the distal end of the first cuff formation, inflation-passage means communicating with both the inflatable cuff formations for selective inflation/deflation operation of the cuff formations; the cuff formations, upon inflation via the inflation-passage means, sealing the airway tube for communication solely with the laryngeal inlet, cushioning a reference to the mask to the posterior wall of the pharynx, and sealing the evacuation tube solely to the sphinctral region; and a reflectance-oximeter probe carried by the second cuff formation and oriented to radiate into and to sense radiation reflected from within a localised portion of the posterior wall of the pharynx.

From another aspect, the invention can be considered to be an artificial airway device to facilitate a patient's lung ventilation, comprising an airway tube, an evacuation tube, and a laryngeal mask at one of the tubes, the mask being of generally elliptical configuration extending from a proximal end to a distal end and in generally a single plane, the configuration being adapted for support by and around the laryngeal inlet and orienting the distal end of the airway tube at an angle to the plane and in substantial alignment with the axis of the laryngeal inlet when supported by and around the laryngeal inlet, the distal end of the mask having a first cuff formation configured for entry into and insertional location of the device by engagement with the upper sphinctral region of the oesophagus when the mask is positioned for airway-tube alignment with the axis of the oesophagus inlet, the evacuation tube having an open distal end centrally within and axially short of the distal end of the first cuff formation, a second inflatable-cuff formation carried by the mask on the posterior side of the plane, inflation-passage means communicating with both cuff formations and a reflectance-oximeter probe carried by the second cuff formation and oriented to radiate into and to sense radiation reflected from within a localised portion of the posterior wall of the pharynx.

Alternatively, the invention can be considered to be in another aspect an artificial airway device to facilitate a patient's lung ventilation, comprising an airway tube, a suction tube, and a laryngeal mask at one end of the tubes, the mask having a proximal end and a distal end and being configured to form a seal of the airway tube solely around the circumference of the patient's laryngeal inlet, the distal end being configured for entry into and insertional location of the device at the entrance of the upper sphinctral region of the oesophagus when the mask is positioned for sealing the airway tube to the laryngeal inlet, the evacuation tube having an open distal end centrally within but axially short of the distal end of the mask, and the distal end of the mask including a first inflatable flexible cuff formation for peripherally sealed engagement of the upper sphinctral region to the distal end of the evacuation tube, thus exposing a passage via the mask for airway-tube communication solely with the laryngeal inlet on the anterior side of the mask, while exposing the posterior side of the mask to the posterior wall of the pharynx, a second inflatable-cuff formation carried by the posterior side of the mask for inflated engagement to the posterior wall of the pharynx, inflation-passage means communicating with both cuff formations, and a reflectance-oximeter probe carried by the second cuff formation and oriented to radiate into and to sense radiation reflected from within a localised portion of the posterior wall of the pharynx.

The invention will now be described in greater detail by way of example only, with reference to the accompanying drawings, in which:
Fig. 1 is a side elevation view showing a laryngeal mask system including an oximeter, with phantom outlines to show features of the head of a patient in whom the mask has been installed;
Fig. 2 is an enlarged posterior-aspect view of the mask of Fig. 1;
Fig. 2A is a sectional view taken along line 2A-2A of Fig. 2;
**Fig. 2B** is a side elevation view of the mask of **Fig. 2**;
**Fig. 3** is a simplified perspective view of a modified mask system, generally as in **Fig. 1,** but with an oximeter-subassembly in exploded readiness for assembly to the mask;
**Fig. 4** is a posterior-aspect view of another modification, wherein a mount for an oximeter is selectively adaptable for and is shown assembled to a laryngeal mask to existing construction;
**Fig. 5** is a perspective view of the oximeter and mount of **Fig. 4**, separate and apart from the mask of **Fig. 4**;
**Fig. 6** is a perspective view of a modified laryngeal mask and oximeter;
**Fig. 7** is a perspective view of a further modified laryngeal mask and oximeter;
**Fig. 8** is a view similar to **Fig. 7**, for the deflated condition of the inflatable components of **Fig. 7**;
**Fig. 9** is a schematic layout of multiple diodes for a modified oximeter-probe portion of a laryngeal mask; and
**Fig. 10** is another schematic layout similar to **Fig. 9** but showing a modification.

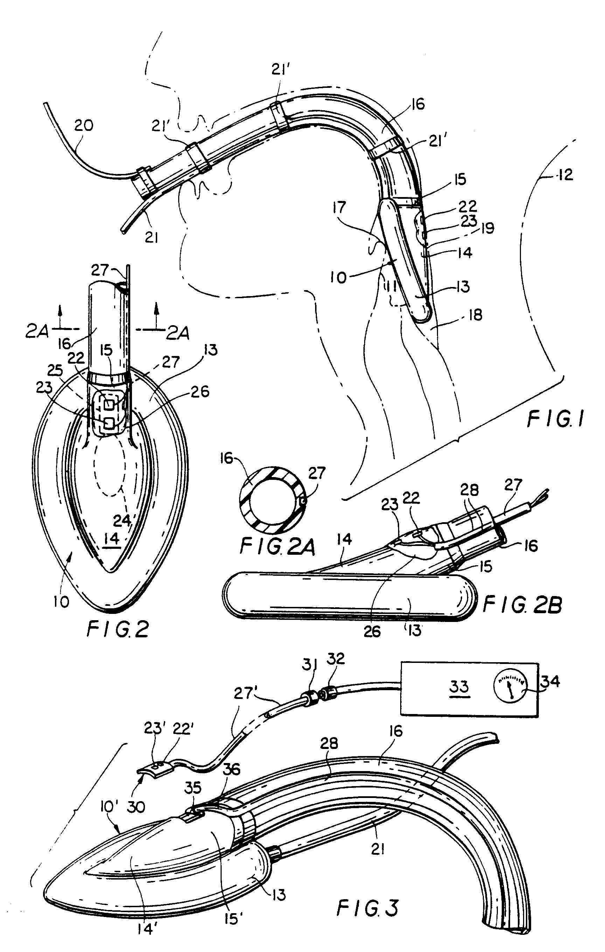

In the installed system of **Fig. 1**, a laryngeal mask 10 has peripherally sealed engagement around the laryngeal inlet 11 of a patient 12. Thus sealed, the mask 10 presents a front (or anterior) side facing into the laryngeal inlet, and a back (or posterior) side facing the back wall of the pharynx. The sealed engagement is via an air-inflated annular ring 13 which is sealed in its connection to a central plate 14. When inflated, ring 13 supports the central plate 14 having an inlet airway port formation 15 on a sloping alignment and at an acute angle with respect to the general plane of ring 13. An elongate airway tube 16 is connected at its distal end to the port formation 15 and is curved for general conformance with the patient's natural breathing passage via the throat to the pharynx. As shown, the seal to the laryngeal inlet surrounds the epiglottis 17 and has a sealed footing at the oesophageal inlet 18; also, the slope of the airway port formation 15 conforms generally with tissues along the posterior wall 19 of the pharynx. The airway tube 16 may be rigid or stiffly flexible, and a manipulating handle 20 is shown at the outer end of tube 16, for facilitating mask insertion into the patient, with the ring 13 in deflated condition, the inflation/deflation procedure being externally controllable via an inflation-air supply tube 21. The particular mask 10 of **Fig. 1** will be understood to be an illustrative one of several varieties, greater detail of which will be found in various of the above-identified patents and patent applications. For reference purposes, the front or anterior side of mask 10 will be understood to be the side which faces the laryngeal inlet passage, and the back or posterior side of mask 10 will be understood to be the side which faces the posterior or back wall 19 of the pharynx.

In accordance with a feature of the invention, oximeter instrumentation is built into or otherwise carried by the mask 10, whereby to enable continuous monitoring of the oxygen content of blood supplied to the brain while also controlling and monitoring air and/or anaesthetic supply to the patient via the tube 16 and mask. In **Fig. 1**, a suggestion of the added oximeter feature appears at the longitudinally spaced diodes 22, 23 which are adhered to the posterior of plate 14, in the region near the air-inlet port formation 15. External wiring which serves diodes 22, 23 has been omitted from **Fig. 1** but will be understood to be preferably retained to or alongside airway tube 16, as by the spaced straps 21' which are shown performing the analogous function for inflation tube 21.

Better detail for oximeter instrumentation 22, 23 carried by mask 10 will be evident from **Figs. 2, 2A** and **2B,** wherein the inflated annular ring 13 is seen to be generally elliptical, with a somewhat pointed distal end, for locating and sealing engagement at the oesophageal inlet. In the plan view of **Fig. 2**, plate 14 is seen to provide an opening 24 in its anterior side for airway communication via airway tube 16 and airway-port formation 15 to the laryngeal inlet. The diodes 22 and 23 may be as provided by **Nellcor Incorporated** (Foster City, San Francisco, California) for their pulse oximeter Model 200, which is commercially available complete with its power supply, signal-generator and reflected-signal detector, and flexible cabling for sufficiently remote connection to the diodes 22, 23; one (22) of these diodes is an LED emitting in the red end of the visible spectrum, while the other (23) is a photodetector diode. In application of such diodes to the present situation, they are advisedly mounted to a thin bed 25 of light absorbing material such as an elastomeric that has been mixed with carbon black to render the same opaque, and this material should be built to greatest thickness between diodes 22, 23, so as to minimize direct coupling of light from the LED to the photodetector. The overall maximum thickness for the diodes in their assembly to each other via a bed 25 is in the order of 2-mm, which is a minimal increase in overall profile for plate 14 and its inlet-port formation 15; the bed 25 may be cemented to formation 15, and an application 26 of self-leveling, optically transparent silicone is sufficient to cover the cemented combination, as well as otherwise-exposed individual insulated electrical leads and cable-sheathing 27 therefor.

As shown in **Figs. 2A** and **2B,** it is preferred that the tubing of airway 16 shall be formed with an elongate outwardly open channel 28 for retaining cable 27; the confronting outer edges of this channel 28 are seen in **Fig. 2B** to be spaced less than the diametral extent of cable 27, thus enabling the cable to be frictionally retained by and to tube 16.

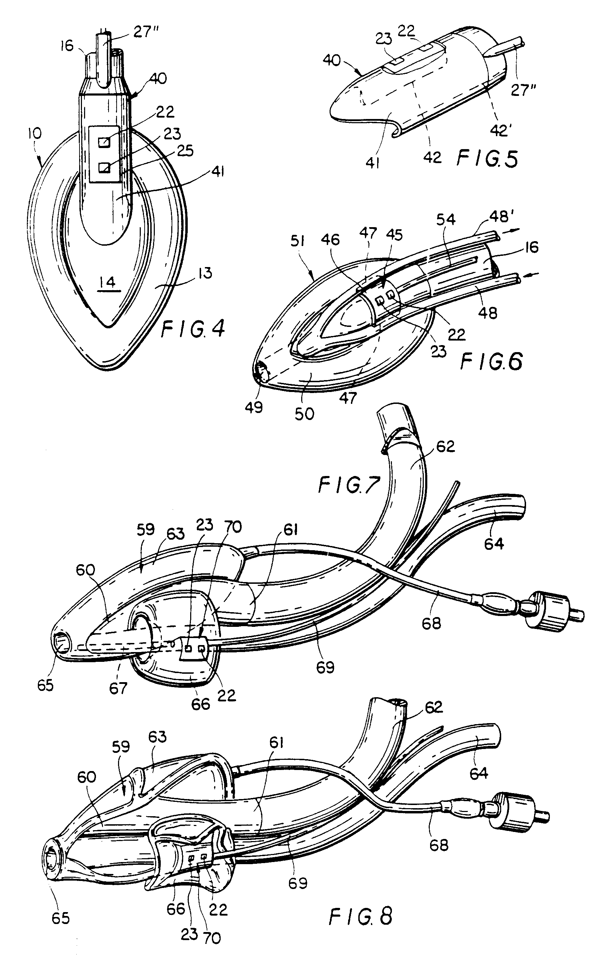

In the embodiment of Fig. 3, the diode or probe unit 30 of oximeter apparatus is a separate article of manufacture, complete with its flexible cabling 27' to an external connector element 31. A second, mating, detachable-connector element 32 is part of a suitable cabinet or housing 33 for signal-generator, signal-processing and display means 34 for monitoring oxygen content of blood observed by the diodes 22', 23' of unit 30. Unit 30 is shown as a thinly developed arcuate block, as of epoxy and having the blackened light-absorbing feature noted above for the devices 22, 23 of **Fig. 2** and their mounting 25. The laryngeal mask 10' of **Fig. 3** will be seen at its region 15' to have been formed with a shallow arcuate recess 35 adapted for removable insertion and retention of diode unit 30, and a shallow groove 36 in the plate-body region 15' communicates with the cable-retaining groove 28 of the airway tube 16, to assure full reception and location of cable 27' all the way from recess 35 to the external region of coupling 31, 32 to the housing 33 of requisite electronics and controls.

In the arrangement of **Figs. 4** and **5**, the unit 40 of **Fig. 5** is also part of a unitary assembly of oximeter-probe components, namely, diode elements 22, 23 locally mounted as previously described, except that the mount 41 is a cylindrically arcuate member which carries the diodes centrally of its upper surface, while its spaced arcuate ends 42, 42' must be compliantly spread in order to be fitted to the customary generally cylindrical profile of the inclined airway-connecting end formation (15) of the mask plate 14. The mount 41 may be of relatively stiff plastic material, or of a relatively softly yieldable plastic material which carries an imbedded cylindrically arcuate stainless-steel spring member (not shown), or the entire mount 41 may be of stainless steel, arcuately formed for clinging, compliantly stressed, retaining engagement to the plate formation 15. Thus, the arrangement of **Figs. 4** and **5** represents a form of the invention which, with its own flexible-lead cable 27'', is removably attachable to an existing laryngeal mask, without requiring any change in the construction of the mask itself.

In the embodiment of **Fig. 6**, a separate unitary oximeter-probe assembly 45 of diodes 22, 23 includes a cylindrically arcuate base 46 with side flanges 47 that removably engage under bifurcated conduit arms 48, 48' for a continuously flowing fluid, such as water or air, or an air/water mixture, for aspirating products of regurgitation from the oesophagus, and entering a sealed passage 49 (to arms 48, 48') through the inflatable ring 50 of a laryngeal mask 51; an in-flow directional arrow in connection with conduit arm 48 and an out-flow directional arrow in connection with conduit arm 48 will be understood to suggest the continuous flow of fluid. Such mask structure is described in greater detail in US patent No. 5 241 956 and therefore needs no present elaboration. It suffices merely to identify the means 48, 48' of such aspiration, piggy-backed to the plate structure 52 and straddling the inclined inlet-port formation 53 of the mask, and to indicate that the probe unit 45 and its flexible-lead cable 54 may be selectively applied to the mask 51.

The mask arrangement of **Figs. 7** and **8** is of another variety described in detail in said US patent application No. 5 241 956. It suffices merely to identify the mask plate 60, its inclined air-inlet port formation 61 (with connected airway tube 62), and its peripherally continuous, elliptically annular inflatable ring 63 for sealed engagement to the laryngeal inlet. A vacuum (i.e., reduced pressure) conduit 64 is piggy-backed to the airway tube and communicates with an opening 65 at the distal end of ring 63, for vacuum extraction and removal of possible products of regurgitation, entering from the oesophagus via opening 65. As disclosed in said US patent No. 5 241 956, a second inflatable/deflatable balloon or cuff 66 surrounds the exposed periphery of conduit 64 at its region of generally central overlap with the mask plate 60 and its inclined air-inlet port formation 61. A single air inflation/deflation line 68 has direct communication with the inflatable laryngeal-seal ring 63 and indirect further communication (via a passage 67, between ring 63 and cuff 66). And a cylindrically arcuate oximeter-probe assembly 70 with spaced diodes 22, 23 is simply adhered to the exterior of cuff 66, in such manner that upon inflation of ring 63 and cuff 66, not only does cuff 66 establish a contour-adapting and stabilizing engagement to the posterior wall of the pharynx (in reacting pressure-loading enhancement of the laryngeal-inlet seal established by the inflated mask ring), but the oximeter-probe unit 70 carried by cuff 66 is firmly applied to the pharyngeal wall, in direct confrontation with the thin tissue of this wall and in relatively closely spaced confronting relation with adjacent vertebra structure that is relied upon for the reflection involved in pulse reflection oximetry.

**Fig. 8** serves to illustrate that upon deflation of ring 63 and cuff 66, not only do the mask (59) components reduce to floppy unimpeding significance, but in addition the oximeter-probe unit 70, being preferably cylindrically arcuate, is drawn into close and nested proximity to the evacuation tube 64, thus presenting no impediment to safe insertion or removal of the mask (with its oximeter-probe unit 70) with respect to its intended situs within the pharynx. Neither does the flexible cabling 69 which serves unit 70 present any difficulty for such insertion or removal of the mask and its oximeter-probe unit.

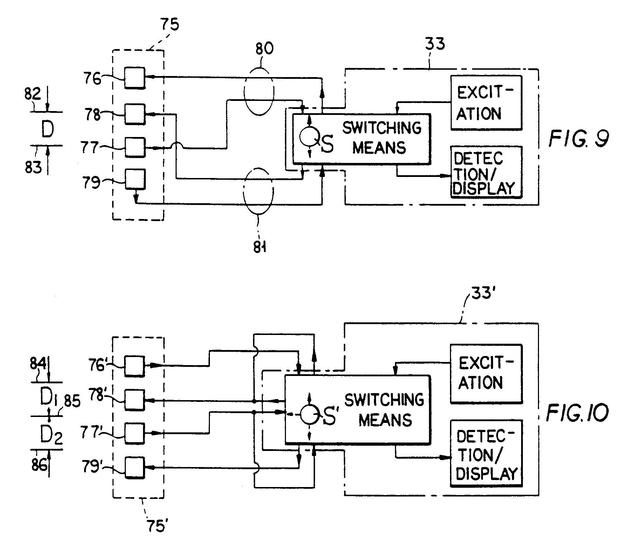

In all of the described embodiments, the oximeter probe has been indicated as involving two diodes, one (22) an LED, and the other (23) a photodiode. Suitable diodes for the indicated purpose are not necessarily square (as schematically shown in the drawings), but their operative areas are in the order of 2 to 4-mm span in their maximum dimension, and it is recommended that they be mounted at approximately 10-mm spacing (center-to-center). Neither is the longtitudinally spaced relation of these diodes necessarily to be preferred, in that optimum results and operation result from good LED radiation through thin pharyngeal tissue thickness and general directional orientation to a good reflecting vertebral surface which will reflect maximally to the photodiodes; such criteria can also be satisfied by a strictly transverse spacing of the diodes 22, 23. Also, as indicated by a diode array schematically indicated in **Fig. 9**, the chances of getting better oximeter performance in any given one of plural patients are improved if an additional two diodes are mounted to their common base 75, in spaced and interlaced array. Thus, in **Fig. 9**, a first pair of diodes (e.g., an LED 76 and its associated photodiode 77), at the recommended center-to-center spacing, may be in longitudinally aligned interlace with a second pair of diodes (e.g., an LED 78 and a photodiode 79). Each coacting pair of diodes may have its own separate lead connections 80 (81) for accommodation in the flexible-cable tie to the external electronics housing, such as housing 33 of **Fig. 3**; and for the convenience of the attending anaesthetist, a manual switch **S** may be available at 33 for his quick selection as to the diode pair to be used for best oximeter performance in a given case. The longitudinal center for the span selection served by diodes 76, 77 is indicated at 82, and the span center for selection of diodes 78, 79 is indicated at 83.

It will be seen that the described embodiments of the invention meet the above-stated objects and also provide certain advantages, including the following:
1. In the United Kingdom, where the laryngeal mask and the pulse oximeter (attached to a finger or toe) are both widely used in general anaesthesia, they are frequently in use at the same time. Attachment of the oximeter to the laryngeal mask therefore eliminates an additional "line" attaching the patient to the anaesthetic trolley, since the cable from the oximeter can follow or even be incorporated in the airway line joining the laryngeal mask to the gas outlet. (There are no explosion hazards with modern anaesthetics). The common problem of a probe being pulled off or falling off a finger or toe is thus elininated.
2. The laryngeal mask is situated in the deepest part of the throat. Therefore an oximeter probe attached in this position will pick up signals of much more relevance to key areas of concern, such as the brain. Moreover, the changes in oxygen saturation will be detected earlier than by use of any peripherally placed oximeter probe.
3. The central position of the oximeter probe and the fact it is in contact with mucosa (lining of the throat) instead of skin both increase the likelihood of obtaining high quality signals, since in the throat, temperature is not significantly different from that of the blood -- thus, no warming is necessary - - and the mucosal blood vessels are in any case closer to the surface.
4. Ambient light interference is no longer a problem, thus eliminating this source of inaccuracy.
5. A further advantage of combining the two devices derives from a unique property of the laryngeal mask: it is the only effective airway device which can safely be left in place in the unconscious patient until full recovery occurs. It is now well known that many problems with breathing (and thus with oxygen uptake) occur during the period of recovery from anaesthesia. An oximeter probe which reliably stays in place during transfer of the patient from operating room to recovery area and maintains constant monitoring until the patient is safely recovered would be much appreciated by anaesthetist and recovery staff.
6. Finally, again in the recovery phase, two factors commonly cause problems with existing peripherally placed oximeter probes: patients may be restless, causing displacement of the probe; and peripheral blood-vessel shutdown may occur,associated with shivering, blood-loss, or pain. Neither of these factors is likely to present a problem with the present invention.

In connection with **Fig. 9**, the plural diodes 76, 78, 77, 79 were described in longitudinally spaced array, as interlaced pairs, so as illustratively to make available the selection of a given pair (76, 77, or 78, 79) for best reflectance; the longitudinal selection thus available with selection of one or the other of these pairs will involve a shift of their respective spans, namely, the distance **D**, shown on the drawing of **Fig. 9.** The same or a similar array will also be seen in **Fig. 10** to provide further alternatives for switching selection at external electronics 33. For example, if diode 78' is an LED, flanked proximally and distally by photodiodes 76', 77', switchable oximeter selection (at **S'**) of diodes 76', 78', or of diodes 77', 78', or of diodes 77', 79' will provide a longitudinal oximeter probe selection as between three spaced locations; the longitudinal center for the span selection served by diodes 76', 78' is indicated at 84, and at 85, 86 for the respective further selectable spans served by diodes 77', 78' and 77', 79', there being equal shifting offsets D₁, D₂ of the center of oximeter measurement for each of the three selectable spans. The selection of one to the exclusion of another longitudinal oximeter location may be by manual switch (**S'**) operation at 33; alternatively, the selection. may be automatic via periodic sampling of the respective alternative oximeter pairs, wherein the samplings are automatically evaluated and switching is automatically effected by known electronic techniques and circuitry, all of which will be understood to be contained within means 33, when connected to leads for optional pairs of diodes, such as shown and discussed in connection with **Figs. 9** and **10**.

The schematic diagrams of **Figs. 9** and **10** will be seen to illustrate electronic circuitry wherein selectively switched use is available for one to the exclusion of other pairs of a plurality (of LED/photodetector pairs). And the case of **Fig. 10** will be seen to illustrate that, when flanked on opposite sides by separate photodetectors (76', 77'), the same LED (78') is effectively continuously connected in switching its paired use with one to the exclusion of the other of the flanking photodetectors (76' 77').

## Claims

1. A laryngeal mask (10,10',51,59) for facilitating lung ventilation, the mask having anterior and posterior sides and means (13,63) comprising for establishing sealed peripheral engagement of the mask around the laryngeal inlet (11) of a patient, with the anterior side facing into the laryngeal inlet and the posterior side facing the back wall (19) of the pharynx, an elongate airway tube (16,62) having a distal end which establishes a sealed passage through the mask between the posterior and anterior sides of the mask, characterized in that a reflectance-oximeter probe (30,40,45,70) is carried at the posterior side of the mask and adapted and oriented to radiate into and to sense radiation reflected from within a localised portion of the back wall of the pharynx.

2. A laryngeal mask according to claim 1, in which the airway tube (16,62) is rigid and curved to follow the airway of a patient.

3. A laryngeal mask according to claim 1 or claim 2, which comprises a plate (14,14',60) via which the airway tube (16,62) has sealed communication with the passage, the plate including a recess (35) and the oximeter probe being carried in the recess.

4. A laryngeal mask according to any one of claims 1 to 3, in which the means for establishing sealed engagement around the laryngeal inlet is an inflatable annular ring (13,63), and wherein the oximeter probe is carried by the mask at a location which, when the mask is viewed in plan, is within the area bounded by the annular ring.

5. A laryngeal mask according to any one of claims 1 to 4, in which the oximeter probe is immovably attached to the posterior side of the mask.

6. A laryngeal mask according to any one of claims 1 to 4, in which the oximeter probe is removably carried on the posterior side of the mask.

7. A laryngeal mask according to any one of claims 1 to 6, in which the mask includes an inflatable cuff (66) at its posterior side for development of distributed-area contact with the back wall of the pharynx, and wherein the oximeter probe is carried by the cuff (66) within the region of cuff contact with the back wall of the pharynx.

8. A laryngeal mask according to claim 7, which includes a single flexible tube (68) connected to the cuff and to the annular ring for communicating inflation air to the cuff and the ring.

9. A laryngeal mask according to any one of claims 1 to 8, in which the oximeter probe includes a flexible cable (27,27',54,69) for establishing external electrical connection to a source of excitation signals for oximeter radiation and to a processor of oximeter-detected signals.

10. A laryngeal mask according to claim 9, in which the airway tube (68) has an elongate local groove (36) adapted to removably retain the cable.

11. A laryngeal mask according to any one of claims 1 to 10, in which the oximeter probe (40) comprises a cylindrically arcuate shell (41) configured for engagement to the airway tube.

12. A laryngeal mask according to any one of claims 1 to 11, in which the oximeter probe comprises an LED (22) in spaced proximity to a photodetector diode (23).

13. A laryngeal mask according to claim 12, in which the spacing of the diodes is longitudinal with respect to the airway-tube.

14. A laryngeal mask according to any one of claims 1 to 11, in which the oximeter probe (75) comprises plural spaced pairs of spaced diode elements, each pair comprising an LED (76,78) and a photodetector diode (77,79).

15. A laryngeal mask according to claim 14, comprising electronic circuitry (33) selectively and remotely switch-connected to one to the exclusion of other pairs of the plurality.

16. A laryngeal mask according to any one of claims 1 to 11, in which the oximeter probe (75) comprises an array of spaced diodes wherein each of two opposite sides of an LED (78') is flanked by a photodetector diode (76',78').

17. A laryngeal mask according to claim 16, comprising electronic circuitry (33') selectively and remotely switch-connected to one to the exclusion of the photodetector diodes while the circuitry is continuously connected to the LED.

## Patentansprüche

1. Kehlkopfmaske (10, 10', 51, 59) zum Erleichtern der Versorgung der Lunge mit Luft, wobei die Maske eine Frontseite und eine Rückseite und eine Einrichtung (13, 63) aufweist, deren Frontseite in den Kehlkopfeingang zeigt und deren Rückseite der Rückwand (19) des Rachens gegenübersteht, und die zum Herstellen eines gedichteten Eingriffs der Maske im Randbereich um den Kehlkopfeingang (11) eines Patienten herum einen langgestreckten Luftwegschlauch (16, 62) mit einem fernen Ende aufweist, das zwischen der Rückseite und der Frontseite der Maske einen gedichteten Durchgang durch die Maske herstellt, dadurch gekennzeichnet, daß ein Reflexionsoximetermeßfühler (30, 40, 45, 70) an der Rückseite der Maske gehalten wird und so ausgelegt und ausgerichtet ist, daß er eine Strahlung in einen räumlich festgelegten Bereich der Rückwand des Rachens abstrahlt und die von diesem Bereich reflektierte Strahlung erfaßt.

2. Kehlkopfmaske nach Anspruch 1, bei welcher der Luftwegschlauch (16, 62) steif und so gekrümmt ist, daß er dem Luftweg eines Patienten folgt.

3. Kehlkopfmaske nach Anspruch 1 oder 2, welche eine Platte (14, 14', 60) enthält, über die der Luftwegschlauch (16, 62) eine gedichtete Verbindung mit dem Durchgang hat, wobei die Platte eine Vertiefung (35) aufweist und der Oximetermeßfühler in der Vertiefung gehalten wird.

4. Kehlkopfmaske nach einem der Ansprüche 1 bis 3, bei welcher die Einrichtung zum Herstellen eines gedichteten Eingriffs um den Kehlkopfeingang herum eine aufblasbare ringförmige Einfassung (13, 63) ist und bei der der Oximetermeßfühler von der Maske an einer Stelle gehalten wird, die bei Betrachtung der Maske in der Draufsicht innerhalb der durch die ringförmige Einfassung begrenzten Fläche liegt.

5. Kehlkopfmaske nach einem der Ansprüche 1 bis 4, bei welcher der Oximetermeßfühler unbeweglich an der Rückseite der Maske befestigt ist.

6. Kehlkopfmaske nach einem der Ansprüche 1 bis 4, bei welcher der Oximetermeßfühler trennbar an der Rückseite der Maske gehalten wird.

7. Kehlkopfmaske nach einem der Ansprüche 1 bis 6, bei welcher die Maske auf der Rückseite eine aufblasbare Manschette (66) zum Herstellen einer sich über eine Fläche erstreckenden Berührung mit der Rückwand des Rachens aufweist und bei welcher der Oximetermeßfühler von der Manschette (66) innerhalb des Bereichs gehalten wird, in der die Manschette in Berührung mit der Rückwand des Rachens steht.

8. Kehlkopfmaske nach Anspruch 7, welche einen einzigen biegsamen Schlauch (68) enthält, der mit der Manschette und der ringförmigen Einfassung zum Übertragen von Aufblasluft zur Manschette und der Einfassung verbunden ist.

9. Kehlkopfmaske nach einem der Ansprüche 1 bis 8, bei welcher der Oximetermeßfühler ein biegsames Kabel (27, 27', 54, 69) zum Herstellen einer externen elektrischen Verbindung zu einer Quelle von Anregungssignalen für die Oximeterstrahlung und zu einer Verarbeitungseinheit für die vom Oximeter erfaßten Signale aufweist.

10. Kehlkopfmaske nach Anspruch 9, bei welcher der Luftwegschlauch (68) eine langgestreckte örtliche Nut (36) aufweist, die für die entfernbare Aufnahme des Kabels ausgelegt ist.

11. Kehlkopfmaske nach einem der Ansprüche 1 bis 10, bei welcher der Oximetermeßfühler (40) eine zylindrisch gebogene Außenwand (41) aufweist, die für den Eingriff in den Luftwegschlauch ausgelegt ist.

12. Kehlkopfmaske nach einem der Ansprüche 1 bis 11, bei welcher der Oximetermeßfühler eine Leuchtdiode (22) enthält, die sich in einem Abstand in der Nähe einer Photodetektordiode (23) befindet.

13. Kehlkopfmaske nach Anspruch 12, bei welcher die Anordnung der Dioden in einem Abstand in Längsrichtung des Luftwegschlauchs verläuft.

14. Kehlkopfmaske nach einem der Ansprüche 1 bis 11, bei welcher der Oximetermeßfühler (75) mehrere in einem Abstand angeordnete Paare in einem Abstand angeordneter Diodenelemente enthält, wobei jedes Paar eine Leuchtdiode (76, 78) und eine Photodetektordiode (77, 79) enthält.

15. Kehlkopfmaske nach Anspruch 14, welche eine elektronische Schaltung (33) enthält, die über einen Schalter wahlweise und aus der Entfernung mit einem aus den mehreren Paaren verbunden wird, wodurch die anderen Paare ausgeschlossen werden.

16. Kehlkopfmaske nach einem der Ansprüche 1 bis 11, bei welcher der Oximetermeßfühler (75) eine lineare Anordnung in einem Abstand angeordneter Dioden enthält, wobei neben jeder der entgegengesetzten Seiten einer Leuchtdiode (78') eine Photodetektordiode (76', 78') liegt.

17. Kehlkopfmaske nach Anspruch 16, welche eine elektronische Schaltung (33') enthält, die über einen Schalter wahlweise und aus der Entfernung mit einer der Photodetektordioden verbunden wird, wobei die anderen ausgeschlossen werden, während die Schaltung fortlaufend mit der Leuchtdiode verbunden ist.

## Revendications

1. Un masque pour le larynx (10, 10', 51, 59) pour faciliter la ventilation des poumons, le masque ayant des côtés antérieur et postérieur et comprenant des moyens (13, 63) pour établir un enclenchement périphérique étanche du masque autour de l'entrée du larynx (11) d'un patient, le côté antérieur faisant face à l'entrée du larynx et le côté postérieur faisant face à la paroi arrière (19) du pharynx, un tube allongé de voie d'air ou de ventilation (16, 62) ayant une extrémité distale qui établit un passage étanche à travers le masque entre les côtés antérieur et postérieur du masque, caractérisé en ce qu'une sonde d'oxymètre à réflexion (30, 40, 45, 70) est portée sur le côté postérieur du masque et adaptée et orientée pour irradier à l'intérieur et pour capter la radiation réfléchie à partir de l'intérieur, d'une partie localisée de la paroi arrière du pharynx.

2. Un masque pour le larynx selon la revendication 1, dans lequel le tube de voie d'air (16, 62) est rigide et courbé pour suivre la voie respiratoire d'un patient.

3. Un masque pour le larynx selon la revendication 1 ou la revendication 2, qui comprend une plaque (14, 14', 60) par l'intermédiaire de laquelle le tube de voie d'air (16, 62) dispose d'une communication étanche avec le passage, la plaque incluant une cavité (35) et la sonde d'oxymètre étant portée dans la cavité.

4. Un masque pour le larynx selon l'une quelconque des revendications 1 à 3, dans lequel les moyens pour établir l'enclenchement étanche autour de l'entrée du larynx est une bague annulaire gonflable (13, 63), et dans lequel la sonde d'oxymètre est portée par le masque à un endroit qui, lorsque le masque est vu en plan, se trouve à l'intérieur de la zone limitée par la bague annulaire.

5. Un masque pour le larynx selon l'une quelconque des revendications 1 à 4, dans lequel la sonde d'oxymètre est fixée à demeure du côté postérieur du masque.

6. Un masque pour le larynx selon l'une quelconque des revendications 1 à 4, dans lequel la sonde d'oxymètre est portée de façon amovible du côté postérieur du masque.

7. Un masque pour le larynx selon l'une quelconque des revendications 1 à 6, dans lequel le masque inclut une manchette gonflable (66) sur son côté postérieur pour développer un contact réparti avec la paroi arrière du pharynx, et dans lequel la sonde d'oxymètre est portée par la manchette (66) à l'intérieur de la région de contact de la manchette avec la paroi arrière du pharynx.

8. Un masque pour le larynx selon la revendication 7, qui inclut un seul tube souple (68) relié à la manchette et à la bague annulaire pour faire passer l'air de gonflage à la manchette et à la bague.

9. Un masque pour le larynx selon l'une quelconque des revendications 1 à 8, dans lequel la sonde d'oxymètre inclut un câble souple (27, 27', 54, 69) pour établir une connexion électrique externe à une source de signaux d'excitation pour la radiation de l'oxymètre et à un processeur des signaux captés par l'oxymètre.

10. Un masque pour le larynx selon la revendication 9, dans lequel le tube de voie d'air (68) présente une rainure locale allongée (36) adaptée pour retenir le câble de façon amovible.

11. Un masque pour le larynx selon l'une quelconque des revendications 1 à 10, dans lequel la sonde d'oxymètre (40) comprend une enveloppe cylindrique arquée (41) qui présente une configuration lui permettant de venir en contact avec le tube de voie d'air.

12. Un masque pour le larynx selon l'une quelconque des revendications 1 à 11, dans lequel la sonde d'oxymètre comprend une diode électrobuninescente DEL (22) à proximité espacée d'une diode de photodétecteur (23).

13. Un masque pour le larynx selon la revendication 12, dans lequel l'espacement des diodes est longitudinal par rapport au tube de voie d'air.

14. Un masque pour le larynx selon l'une quelconque des revendications 1 à 11, dans lequel la sonde d'oxymètre (75) comprend une pluralité de paires espacées d'éléments de diode espacés, chaque paire comprenant une DEL (76, 78) et une diode de photodétecteur (77, 79).

15. Un masque pour le larynx selon la revendication 14, comprenant des circuits électroniques (33) connectés par commutateur, sélectivement et à distance, à une paire, à l'exclusion des autres paires, de la pluralité.

16. Un masque pour le larynx selon l'une quelconque des revendications 1 à 11, dans lequel la sonde d'oxymètre (75) comprend un agencement de diodes espacées dans lequel chacun des deux côtés opposés d'une DEL (78') est encadré par une diode de photodétecteur (76', 78').

17. Un masque pour le larynx selon la revendication 16, comprenant des circuits électroniques (33') connectés par commutateur, sélectivement et à distance, à une diode à l'exclusion des diodes de photodétecteur tandis que les circuits sont continuellement connectés à la DEL.
